# EUROPÄISCHE PATENTANMELDUNG

(11) **EP 2 918 264 A1**
(43) Veröffentlichungstag der Anmeldung: **16.09.2015**
(21) Anmeldenummer: 14159749.2
(22) Anmeldetag: 14.03.2014
(51) Int. Cl.: A61K 9/00, A61K 9/14, A61K 9/50, B82Y 5/00, B82Y 30/00, C08J 3/12

(54) **Polymere Nanopartikelformulierungen mit maskierter Oberfläche zum Schutz des pulmonalen Surfactants**

(71) Anmelder: Justus-Liebig-Universität Gießen, 35390 Gießen (DE); Transmit Gesellschaft für Technologietransfer mbH, 35394 Giessen (DE)
(72) Erfinder: Schmehl, Thomas, 35390 Giessen (DE); Beck-Broichsitter, Moritz, 92290 Châtenay-Malabry (FR)
(74) Vertreter: Stumpf, Peter

(57) **Zusammenfassung**

Die Nanotechnologie bietet eine Vielzahl neuer Materialien mit umfangreichen Möglichkeiten potenzieller Anwendungen im Bereich der Medizin. In den letzten Jahren wurden nanoskalige Materialien als mögliche Arzneistoffträger, auch für die pulmonale Applikation, untersucht. Es ist jedoch bekannt, dass unbeschichtete Nanopartikelformulierungen das pulmonale Surfactantsystem inhibieren können. Die vorliegende Erfindung betrifft deshalb eine polymere Nanopartikelformulierung mit einem Durchmesser zwischen 10 nm und 1000 nm, wobei die polymere Nanopartikelformulierung im Inneren einen Kern aufweist und der Kern wenigstens ein Kern-Polymer umfasst und wobei auf der Oberfläche des Kerns eine maskierende Beschichtung vorgesehen ist, die Oberflächenbeschichtung ein Beschichtungs-Polymer umfasst und eine Schichtdicke von 1 bis 20 nm aufweist. Die polymeren Nanopartikelformulierungen sind in der Lage, eine niedrige Oberflächenspannung in der Lunge aufrecht zu erhalten und den pulmonalen Surfactant zu schützen.

## Beschreibung

Die vorliegende Erfindung betrifft polymere Nanopartikelformulierungen mit einem Durchmesser zwischen 10 nm und 1000 nm, wobei die polymeren Nanopartikelformulierungen im Inneren einen Kern aufweisen und der Kern wenigstens ein Kern-Polymer umfasst. Die Nanopartikelformulierungen üben keinerlei schädliche Wirkungen auf das essentielle pulmonale Surfactantsystem der Lunge aus.

### Stand der Technik

Nanoskalige Materialien stellen potentielle Arzneistoffträgersysteme für die pulmonale Applikation dar (Beck-Broichsitter M, Merkel OM, Kissel T: Controlled pulmonary drug and gene delivery using polymeric nano-carriers. J Controlled Release 2012, 161:214-224.). Dies betrifft beispielsweise polymere Nanopartikelformulierungen, die zur Entwicklung verbesserter Behandlungsansätze respiratorischer Erkrankungen beitragen. Mit Hilfe der direkten Applikation verkapselter Wirkstoffe in die Lunge kann eine lang anhaltende und kontrollierte Wirkstofffreigabe am gewünschten Wirkort erreicht werden, was zu einer verlängerten pharmakologischen Wirkung führt (Beck-Broichsitter M, Gauss J, Packhaeuser CB, Lahnstein K, Schmehl T, Seeger W, Kissel T, Gessler T: Pulmonary drug delivery with aerosolizable nanoparticles in an ex vivo lung model. Int J Pharm 2009, 367:169-178; Beck-Broichsitter M, Gauss J, Gessler T, Seeger W, Kissel T, Schmehl T: Pulmonary targeting with biodegradable salbutamol-loaded nanoparticles. J Aerosol Med 2010, 23:47-57; Rytting E, Bur M, Cartier R, Bouyssou T, Wang X, Krueger M, Lehr CM, Kissel T: In vitro and in vivo performance of biocompatible negatively-charged salbutamolloaded nanoparticles. J Controlled Release 2010, 141:101-107; Roa WH, Azarmi S, Al-Hallak MHDK, Finlay WH, Magliocco AM, Loebenberg R: Inhalable nanoparticles, a non-invasive approach to treat lung cancer in a mouse model. J Controlled Release 2011, 150:49-55; Trivedi R, Redente EF, Thakur A, Riches DWH, Kompella UB: Local delivery of biodegradable pirfenidone nanoparticles ameliorates bleomycin-induced pulmonary fibrosis in mice. Nanotechnology 2012, 23:505101.).

Der Alveolarraum von Säugerlungen ist von einem komplexen Surfactantsystem bedeckt, das die Oberflächenspannung reduziert, um ein Kollabieren der Alveolen während des Atmens zu verhindern (Perez-Gil J: Structure of pulmonary surfactant membranes and films: The role of proteins and lipid-protein interactions. BBA-Biomembranes 2008, 1778:1676-1695; Possmayer F, Hall SB, Haller T, Petersen NO, Zuo YY, Bernardino de la Serna J, Postle AD, Veldhuizen RAW, Orgeig S: Recent advances in alveolar biology: Some new looks at the alveolar interface. Respir Physiol Neurobiol 2010, 173S:S55-S64.). Pulmonaler Surfactant enthält ungefähr 90 % Lipide und 10 % Surfactant-assoziierte Proteine (SP). Die Lipide, die die Alveolaroberflächen bedecken, bestehen vor allem aus Phospholipiden (PL) (-80-90 %) und einem kleineren Anteil neutraler Lipide (~10-20 %). Unter den Phospholipiden überwiegen Phosphatidylcholine (~70-80 %) und Phosphatidylgycerole. Das pulmonale Surfactant ist ein wichtiger Bestandteil des Alveolarraums der Lunge und für die Regulation der Oberflächenspannung der endständigen Lufträume bedeutend, wodurch der Gasaustausch gefördert wird.

Etwa die Hälfte der Proteinmasse des alveolären Surfactants besteht aus den Surfactant-assoziierten Proteinen SP-A und SP-D, bei denen es sich um hochmolekulare hydrophile Proteine handelt, sowie SP-B und SP-C, die niedermolekulare hydrophobe Proteine sind. Zahlreiche *in vitro*-Studien beschäftigten sich mit der komplexen Wechselwirkung zwischen Phospholipiden (Phosphatidylcholinen und Phosphatidylglycerolen) und Surfactantproteinen (SP-B und SP-C), die die Abnahme der Oberflächenspannung in der Alveolarregion auf Werte nahe 0 mN/m während der Kompressions-Expansions-Zyklen ermöglichen. Während der Kompression des Oberflächenfilms (Exspiration) fördern SP-B und SP-C die Reinigung der Monoschicht. Bei Expansion der Alveolaroberfläche (Inspiration) erleichtern SP-B und SP-C den schnellen Wiedereintritt und die Wiederausbreitung der Surfactantlipide, was für die Limitierung des Anstiegs der Oberflächenspannung essentiell ist.

Faktoren mit unerwünschten Wirkungen auf die Funktion des Surfactantsystems können folglich erhebliche Konsequenzen haben (Zuo YY, Veldhuizen RAW, Neumann AW, Petersen NO, Possmayer F: Current perspectives in pulmonary surfactant - Inhibition, enhancement and evaluation. BBA-Biomembranes 2008, 1778:1947-1977.)

Gemäß der WO 2012/010159 A1 sind biokompatible Nano-, Meso- und Mikropolymerpartikel zur Bindung pathogener Proteine, die in den Lining Layer der Lunge eindringen, bekannt. Die Partikel weisen danach einen Durchmesser zwischen 20 nm und 10 µm auf, sind wasserunlöslich, haben eine positive Oberflächenladung, weisen eine geringe Oberflächenhydrophobizität auf, besitzen einen isoelektrischen Punkt, der größer als 5 ist und der zugleich größer ist als der isoelektrische Punkt des zu bindenden pathogenen Proteins und besitzen ein positives ζ-Potential von größer als +20 mV. Werden derartige Polymerpartikel in die Lunge eingebracht können diese durch Adsorption von in den Lining Layer der Lunge eingedrungenen Plasmaproteinen die pulmonale Surfactant-Funktion positiv beeinflussen. Adsorbierte Plasmaproteine sind nicht mehr in der Lage, die Struktur des Surfactants an der Luft-Wasser-Grenzfläche zu stören.

Es ist jedoch bekannt, dass unbeschichtete Nanopartikelformulierungen das pulmonale Surfactantsystem inhibieren können (Beck-Broichsitter M, Ruppert C, Schmehl T, Guenther A, Betz T, Bakowsky U, et al. Biophysical investigation of pulmonary surfactant surface properties upon contact with polymeric nanoparticles in vitro. Nanomedicine: NBM 2011, 7:341-350; Beck-Broichsitter M, Ruppert C, Schmehl T, Günther A, Seeger W. Biophysical inhibition of synthetic vs. naturally-derived pulmonary surfactant preparations by polymeric nanoparticles. BBA-Biomembranes 2014, 1838:474-481.).

Aufgrund der Bedeutung polymerer Nanopartikelformulierungen für die pulmonale Applikation besteht ein erheblicher Bedarf an Arzneistoffträgersystemen, die eine verringerte unerwünschte Wirkung auf das essentielle pulmonale Surfactantsystem haben.

### Aufgabe

Aufgabe der Erfindung ist es, polymere Nanopartikelformulierungen zur Aufrechterhaltung einer niedrigen Oberflächenspannung in der Lunge bereitzustellen.

### Lösung der Aufgabe

Die oben genannte Aufgabe wird erfindungsgemäß gelöst durch die in den Patentansprüchen charakterisierten Ausführungsformen.

Nach einer ersten Ausführungsform bezieht sich die vorliegende Erfindung auf eine polymere Nanopartikelformulierung mit einem Durchmesser zwischen 10 nm und 1000 nm, wobei die Nanopartikelformulierung im Inneren einen Kern aufweist und der Kern wenigstens ein Kern-Polymer umfasst und wobei auf der Oberfläche des Kerns eine maskierende Beschichtung vorgesehen ist, die Oberflächenbeschichtung ein Beschichtungs-Polymer umfasst und eine Schichtdicke von 1 bis 20 nm aufweist. Die erfindungsgemäßen polymeren Nanopartikelformulierungen umfassen mit anderen Worten im Inneren einen Kern, der aus wenigstens einem Kern-Polymer gebildet wird, und auf der Oberfläche zum Zweck der Maskierung eine Schicht aus einem Beschichtungs-Polymer. Diese polymere Nanopartikelformulierung hat den Vorteil, dass die Oberflächenbeschichtung eine Schutzschicht um den Nanopartikelkern bildet.

Überraschend wurde gefunden, dass die erfindungsgemäßen polymeren Nanopartikelformulierungen in der Lage sind, eine niedrige Oberflächenspannung in der Lunge aufrecht zu erhalten und den pulmonalen Surfactant dadurch zu schützen.

Nach einem weiteren Aspekt der Erfindung können unerwünschte Wechselwirkungen der Nanopartikel bei pulmonaler Applikation mit dem pulmonalen Surfactantsystem vermieden oder verringert werden. Weiterhin kann eine Adsorption von Komponenten des pulmonalen Surfactantsystems an Nanopartikel vermindert werden. Weiterhin kann eine schädigende Auswirkung von Nanopartikeln auf die Menge oder die Zusammensetzung der Surfactant-assoziierten Proteine des alveolären Surfactants vermieden oder verringert werden.

Die Schichtdicke der Oberflächenbeschichtung von 1 bis 20 nm ist erforderlich, um die Wechselwirkung mit Surfactantbestandteilen zu minimieren.

Im Sinne der vorliegenden Erfindung wird unter "Nanopartikel" jeder polymere Partikel mit einer Größe zwischen 10 nm und 1000 nm verstanden.

Mit "Nanopartikelformulierung" wird, wenn dies nicht ausdrücklich anders gekennzeichnet ist, vorliegend ein beschichteter Nanopartikel bezeichnet.

Die Begriffe "maskierende Beschichtung" und "Oberflächenbeschichtung" werden hier synonym verwendet.

Gemäß einer weiteren Ausführungsform der erfindungsgemäßen polymeren Nanopartikelformulierung ist das Beschichtungs-Polymer der Oberflächenbeschichtung kovalent oder adsorptiv auf den Kern aufgebracht. Die adsorptive Aufbringung hat den Vorteil, dass eine flexible Kombination aus Kern-Polymer und Beschichtungs-Polymer möglich ist.

Nach einer weiteren Ausführungsform der erfindungsgemäßen polymeren Nanopartikelformulierung weist die Oberflächenbeschichtung eine Schichtdicke von 2 bis 10 nm, vorzugsweise 4 bis 10 nm und weiter bevorzugt 5 bis 8 nm auf. Diese Schichtdicken erlauben eine Verringerung der Wechselwirkung mit Surfactantbestandteilen.

Die erfindungsgemäßen polymeren Nanopartikelformulierungen können nach einer vorteilhaften Abwandlung bei Bedarf eine Größe von 20 nm bis 500 nm, vorzugsweise von 100 nm bis 200 nm aufweisen. Dem Fachmann ist bekannt, dass die Größe der Partikel je nach vorgesehener Anwendung anzupassen ist.

Eine erfindungsgemäße Weiterentwicklung betrifft polymere Nanopartikelformulierungen, die ein ζ-Potential größer als -40 mV, also ein weniger negatives als -40 mV oder ein positives ζ-Potential, aufweisen. Die polymeren Nanopartikelformulierungen weisen vorzugsweise ein ζ-Potential größer als -30 mV auf. Idealerweise sollte das ζ-Potential möglichst nah an 0 mV liegen, unabhängig davon, ob entsprechende unbeschichtete Nonopartikel ein negatives oder positives ζ-Potential aufweisen. Die polymeren Nanopartikelformulierungen weisen somit vorzugsweise ein ζ-Potential von im Wesentlichen nahe 0 mV auf, wobei eine Annäherung an 0 mV angestrebt wird. Die maskierende Oberflächenbeschichtung mit dem Beschichtungs-Polymer hat dabei den Vorteil einer Änderung des ζ-Potentials im Vergleich zu einem unbeschichteten polymeren Nanopartikel, wobei im Idealfall eine Annäherung an nahe 0 mV erzielt wird. Diese Anpassung des ζ-Potentials auf Grund der maskierenden Oberflächenbeschichtung trägt zu einer verringerten Adsorption von Komponenten des pulmonalen Surfactantsystems an die beschichteten Nanopartikel bei.

Gemäß einer weiteren Ausführungsform der erfindungsgemäßen polymeren Nanopartikelformulierungen ist das Kern-Polymer ausgewählt aus der Gruppe der Homopolymere, Copolymere, Blockpolymere, Blockcopolymere, Pfropfcopolymere, Sternpolymere, Kammpolymere, hochverzweigten Polymere, statistischen Polymere, Dendrimere und deren Mischungen. Das Kern-Polymer ist vorzugsweise in der Art gewählt, dass es eine Beladung mit einem Wirkstoff oder mit einem Diagnostikum erlaubt. Dem Fachmann sind derartige Polymere bekannt.

Das Kern-Polymer ist vorzugsweise ein biokompatibles, bioabbaubares Polymer. Gemäß einer weiteren Ausführungsform der erfindungsgemäßen polymeren Nanopartikelformulierung ist ein biokompatibles, bioabbaubares Kern-Polymer ausgewählt aus der Gruppe der Polyester, Poly(dioxanon)e, Poly(hydroxybutyrat)e, Poly(cyanoacrylat)e, Poly(amid)e, Poly(urethan)e, Poly(organophosphazen)e, Polyanhydride, Polyorthoester, Polycarbonate, Polyketale, Proteine, oder Polysaccharide. Dem Fachmann sind weitere geeignete biokompatible, bioabbaubare Polymere bekannt, wie etwa Polyphosphorester, Polyharnstoffe oder Polyether-Polyester Blockcopolymer. Wenn dies zweckdienlich ist, ist das Kern-Polymer wasserunlöslich. Die Materialien für die Nano- und Mikropartikel sind vorzugsweise für die pulmonale Applikation geeignet.

Bevorzugte Kern-Polymere sind Polyester, wie beispielsweise eine Polymilchsäure, insbesondere Poly(D,L-lactid) (PLA), oder ein Polylactid-co-Glycolid, insbesondere Poly(D,L-laktid-co-glykolid)-Copolymer (PLGA).

Nach einer weiteren Ausführungsform der erfindungsgemäßen polymeren Nanopartikelformulierung ist das Beschichtungs-Polymer der Oberflächenbeschichtung ausgewählt aus der Gruppe der Homopolymere, Copolymere, Blockpolymere, Blockcopolymere, Pfropfcopolymere, Sternpolymere, Kammpolymere, hochverzweigten Polymere, statistischen Polymere, Dendrimere und deren Mischungen.

Das Beschichtungs-Polymer ist vorzugsweise in der Art gewählt, dass eine adsorptive oder kovalente Oberflächenbeschichtung des Kern-Polymers möglich ist. Weiterhin sollte das Beschichtungs-Polymer unter physiologischen Bedingungen im Alveolarraum der Lunge vorzugsweise, jedenfalls zu einem gewissen Anteil löslich, sein. Demnach ist das Beschichtungs-Polymer bevorzugt teilweise oder insgesamt amphiphil/hydrophil.

Nach einer vorteilhaften Ausführungsform umfasst das Beschichtungs-Polymer ein Tensid, insbesondere ein Poloxamer. Als besonders vorteilhaft haben sich Poloxamere als Polymer der Oberflächenbeschichtung herausgestellt. Poloxamere sind Copolymerisate (Blockcopolymere) aus Polyethylenglykolen und Polypropylenglykolen der allgemeinen Formel HO-[(CH₂)₂-O]ₐ-[(CH₂)₃-O-]_{b}-[(CH₂)₂-O]ₐ-H. Dabei ist a = 2-130 und b = 15-67. Der Polyethylenoxidteil des Polymers ist dabei wasserlöslich. Die für das jeweilige Poloxamer angegebene Nummer hat dabei folgende Bedeutung: Die ersten beiden Ziffern geben mit 100 multipliziert das durchschnittliche Molekulargewicht des Poly(oxypropylen)-Anteils an. Die letzte Ziffer gibt mit 10 multipliziert den Gewichtsprozentgehalt des Poly(oxyethylen)-Anteils an. Das Poloxamer kann im Sinne der vorliegenden Erfindung vorzugsweise Poloxamer 407, Poloxamer 188, Poloxamer 338, Poloxamer 181 sein. Dem Fachmann sind weitere geeignete Poloxamere bekannt.

Nach einer weiteren vorteilhaften Ausführungsform umfasst das Beschichtungs-Polymer beispielsweise ein Polyether wie Polyethylenglycol (PEG).

Ein weiterer Aspekt der vorliegenden Erfindung betrifft polymere Nanopartikelformulierungen nach einer oben beschriebenen Ausführungsform, wobei die polymeren Nanopartikelformulierungen mit wenigstens einem Wirkstoff und/oder Diagnostikum beladen sind. Dabei sind der Wirkstoff und/oder das Diagnostikum im Inneren der polymeren Nanopartikelformulierungen, also im Bereich des Kerns, zu finden.

Nanoskalige Materialien als mögliche Arzneistoffträger, auch für die pulmonale Applikation, sind dem Fachmann bekannt. Dies betrifft beispielsweise spezielle polymere Nanopartikelformulierungen, die zur Entwicklung verbesserter Behandlungsansätze respiratorischer Erkrankungen beitragen.

Vorzugsweise enthält die polymere Nanopartikelformulierung einen zur pulmonalen Applikation geeigneten Wirkstoff. Dem Fachmann sind dazu vorgesehene Wirkstoffe bekannt. Die Wirkstoffe können für eine systemische Therapie oder auch eine lokale Therapie vorgesehen sein. Entscheidend für die Wirkung der erfindungsgemäßen polymeren Nanopartikelformulierung ist nach einem Aspekt der Erfindung lediglich die Route der Applikation, nämlich über die Lunge. Bisher waren toxikologische Aspekte der Wechselwirkung von Polymernanopartikeln (NP) mit dem pulmonalen Surfactantsystem bekannt. Unerwünschte Wirkungen auf die Funktion des Surfactantsystems durch Polymernanopartikeln können erhebliche Konsequenzen haben. Diese unerwünschten Wirkungen von Polymernanopartikeln auf die Funktion des Surfactantsystems können durch die erfindungsgemäßen polymeren Nanopartikelformulierungen verringert oder vermieden werden. Die Bedeutung nanoskaliger Materialien als Arzneistoffträger, insbesondere für die pulmonale Applikation verkapselter Wirkstoffe in die Lunge, lässt die breiten Anwendungsmöglichkeiten der hier zur Verfügung gestellten polymeren Nanopartikelformulierung erkennen.

Die erfindungsgemäßen polymeren Nanopartikelformulierungen sind vorteilhaft mit piezoeleketrischen, Düsen-, Ultraschall-Aerosolgeneratoren, Soft-Mist-Inhalern, Metered Dose Inhalern oder Dry Powder Inhalern vernebelbar, d.h. die Applikation in die Lunge erfolgt durch Inhalation eines Aerosols (Suspension, Pulver) mit Hilfe eines Verneblers.

Als eine selektivere Route der Verabreichung pulmonaler Wirkstoffe ist dem Fachmann die Inhalation bekannt, wodurch unerwünschte systemische Nebenwirkungen umgangen werden können. Die direkte Applikation des Wirkstoffs in die Lunge erleichtert die gezielte Behandlung respiratorischer Erkrankungen.

Eine weitere Möglichkeit der Applikation in die Lunge besteht in der Instillation, beispielsweise über einen Katheter, ein Bronchoskop oder einen Beatmungszugang (z.B. Tubus oder Trachealkanüle).

Ein weiterer Aspekt der vorliegenden Erfindung betrifft ein Arzneimittel enthaltend eine polymere Nanopartikelformulierung gemäß einem der oben dargestellten Ausführungsformen. Dazu ist die polymere Nanopartikelformulierung vorzugsweise mit einer therapeutisch wirksamen Menge an Wirkstoff beladen.

Die erfindungsgemäßen polymeren Nanopartikelformulierungen sind insbesondere zur Verwendung bei der Behandlung von respiratorischen Erkrankungen vorgesehen. Man erkennt, dass durch die Oberflächenbeschichtung der erfindungsgemäßen polymeren Nanopartikelformulierung unerwünschte Wechselwirkungen der Nanopartikel, insbesondere bei pulmonaler Applikation, mit dem pulmonalen Surfactantsystem vermieden oder verringert werden können. Die erfindungsgemäßen polymeren Nanopartikelformulierungen sind in der Lage, eine niedrige Oberflächenspannung in der Lunge aufrecht zu erhalten und damit den pulmonalen Surfactant zu schützen. Insbesondere kann eine schädigende Auswirkung von Nanopartikeln auf die Menge oder die Zusammensetzung der Surfactant-assoziierten Proteine des alveolären Surfactants vermieden oder verringert werden.

Nach einem weiteren Aspekt der vorliegenden Erfindung sind polymere Nanopartikelformulierungen gemäß einer der dargestellten Ausführungsformen vorgesehen zur Verwendung zur Prävention und/oder Behandlung von an einer respiratorischen Erkrankung leidenden Patienten, insbesondere solcher Erkrankungen, die mit einem Anstieg der Oberflächenspannung in der Lunge und einer Schädigung des pulmonalen Surfactants einhergehen.

Nach einem weiteren Aspekt der vorliegenden Erfindung sind polymere Nanopartikelformulierungen gemäß einer der dargestellten Ausführungsformen vorgesehen zur Verwendung zur Prävention und/oder Behandlung von an einer respiratorischen Erkrankung leidenden Patienten, wobei nach pulmonaler Verabreichung der polymeren Nanopartikelformulierung die minimale Oberflächenspannung (γₘᵢₙ Werte) des pulmonalen Surfactant-Systems des Patienten kleiner als 10 mN/m, vorzugsweise kleiner als 5 mN/m, und/oder die Konzentration von Surfactant-assoziierten Proteinen (SP) höher als 0,1 Gew.-%, vorzugsweise höher als 0,2 Gew.-%, besonders bevorzugt höher als 0,5 Gew.-%, ist. Man erkennt, dass im Sinne dieser Ausführungsform, die mit der pulmonalen Verabreichung von Nanopartikeln bisher verbundenen unerwünschten Nebenwirkungen auf das Surfactantsystem verringert bzw. vermieden werden.

Der Patient ist im Sinne der vorliegenden Erfindung ein Säugetier und insbesondere ein Mensch.

Nach einem weiteren Aspekt der vorliegenden Erfindung sind polymere Nanopartikelformulierungen gemäß einer der dargestellten Ausführungsformen vorgesehen zur Verwendung zur Behandlung von an einer respiratorischen Erkrankung leidenden Patienten, wobei die Patienten ausgewählt sind aus einer Gruppe, die dadurch charakterisiert ist, dass die minimale Oberflächenspannung (γₘᵢₙ Werte) des pulmonalen Surfactant-Systems des Patienten größer als 5 mN/m, vorzugsweise größer als 10 mN/m, und/oder die Konzentration von Surfactant-assoziierten Proteinen (SP) niedriger als 0,2 Gew.-%, vorzugsweise niedriger als 0,1 Gew.-%, ist. Man erkennt, dass im Sinne dieser Ausführungsform, insbesondere ein bereits geschädigtes Surfactantsystem eines Patienten behandelt werden kann werden.

Ein weiterer Aspekt betrifft Verwendungen einer erfindungsgemäßen polymeren Nanopartikelformulierung zur Behandlung von respiratorischen Erkrankungen gemäß einer der oben genannten Ausführungsformen.

Die erfindungsgemäßen polymeren Nanopartikelformulierungen können zur Herstellung eines pharmazeutischen Mittels zur Prävention und/oder Behandlung von Lungenerkrankungen im Sinne einer der oben genannten Verwendungen genutzt werden, insbesondere solcher Erkrankungen, die mit einem Anstieg der Oberflächenspannung in der Lunge und einer Schädigung des pulmonalen Surfactants einhergehen. Die erfindungsgemäßen Polymerpartikel dienen dabei der Wiederherstellung und Aufrechterhaltung einer niedrigen Oberflächenspannung in der Lunge und dem Schutz des pulmonalen Surfactants.

Die Lungenerkrankungen im Sinne der vorliegenden Erfindung sind vorzugsweise ausgewählt aus der Gruppe bestehend aus Neonatal Respiratory Distress Syndrome, Acute/Adult Respiratory Distress Syndrome (ARDS), Acute Lung Injury (ALI), Lungeninfektionen, Pneumonie, pulmonale Hypertonie, kardiogenes Lungenödem, Asthma, Chronic Obstructive Pulmonary Disease (COPD) / Emphysem, interstitielle Lungenerkrankungen, Lungentumoren, toxische Alveolitis, alveoläres Hämorrhagie-Syndrom, Mukoviszidose, idiopathische pulmonale Hämosiderose, Kollagenkrankheiten, Vaskulitiden, Pneumokoniosen, eosinophile Lungeninfiltrate, Strahlenschäden, hereditäre oder kongenitale Lungenerkrankungen.

Ein weiterer Aspekt der vorliegenden Erfindung betrifft polymere Nanopartikelformulierung nach einer oben beschriebenen Ausführungsform, wobei die polymere Nanopartikelformulierung mit wenigstens einer geeigneten Substanz zur Diagnostik beladen ist. Dabei kann diese Substanz entweder im Inneren der polymeren Nanopartikelformulierung, also im Bereich des Kerns, oder in der Oberflächenbeschichtung oder in beiden Bestandteilen vorgesehen sein. Bei einer geeigneten Substanz zur Diagnostik kann es sich sowohl um *in vitro-* als auch um *in* vivo-Diagnostika handeln. Ein erfindungsgemäß einzusetzendes Diagnostikum kann beispielsweise bildgebend und/oder radioaktiv und/oder ein Kontrastmittel sein. Man erkennt, dass bei Einsatz der polymeren Nanopartikelformulierungen zum Zweck der Diagnostik eine unerwünschte Beeinflussung der Oberflächenspannung und des pulmonalen Surfactants in der Lunge vermieden werden kann.

### Ausführungsbeispiele

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen.

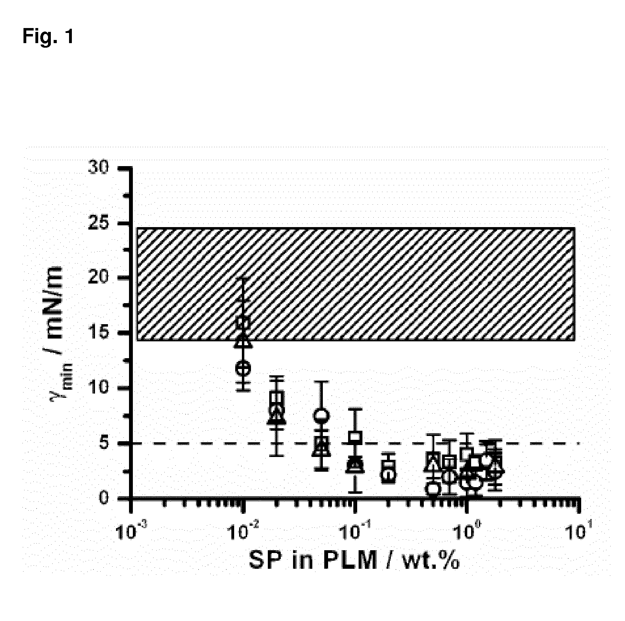

- Fig. 1: Das Diagramm zeigt in den verschiedenen PLM Präparationen mit Verringerung des SP Gehalts einen Dosis-abhängigen Anstieg der γₘᵢₙ Werte für die Oberflächenspannung bewirkt. Dabei sind die γₘᵢₙ Werte in mN/m für die Surfactant-assoziierten Proteine SP-B (offene Quadrate), rSP-C (offene Kreise) und SP-B/C (offene Dreiecke) im jeweiligen PLM dargestellt. Die Werte sind als Mittel mit Standardabweichung (n=5) angegeben. Das eingegrenzte Rechteck gibt das Mittel mit Standardabweichung für reines PLM wieder. (Vergleichsbeispiel)

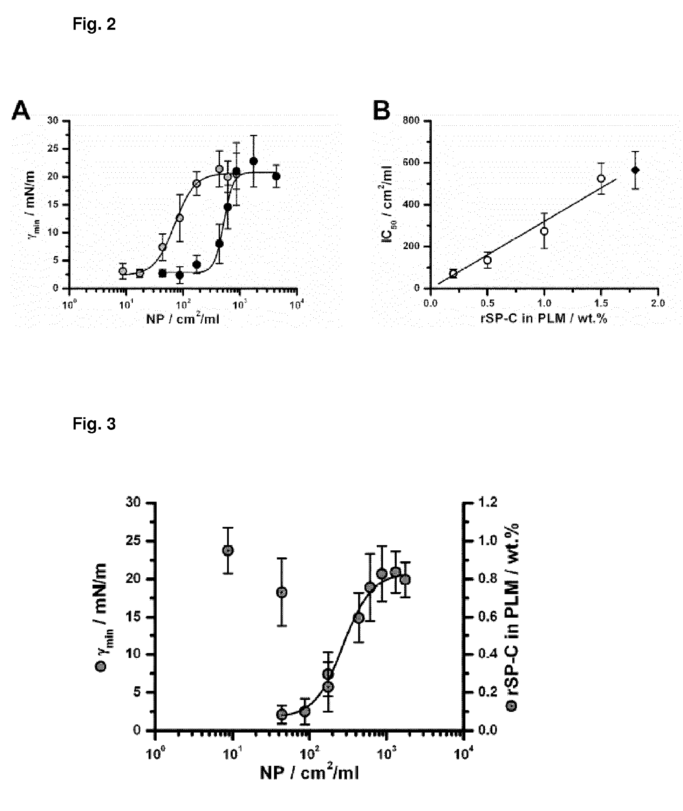

- Fig. 2A: Das Diagramm zeigt eine Dosis abhängige Wirkung von PLA-NP auf die γₘᵢₙ Werte des mit 0,2 Gew.-% (hellgraue Kreise) bzw. 1,5 Gew.-% (dunkle Kreise) rSP-C versetzten des PLM hervor. (Vergleichsbeispiel)
- Fig. 2B: Ein Zusammenhang zwischen der rSP-C Konzentration in PLM und der PLA-NP induzierten biophysikalischen Inhibition ist gezeigt, wobei die IC₅₀ Werte aus der sigmoidale Dosis-Wirkung gemäß Fig. 2A errechnet wurden. Das Maß der aus den Dosis-Wirkung Kurven gemäß Fig. 2A in individuellen Inhibitions-Ansätzen abgeleiteten biophysikalischen Inaktivierung (IC₅₀) ist in einem Diagramm gegen die Menge des im PLM anwesenden rSP-C aufgetragen. (Vergleichsbeispiel)
- Fig. 3: Das Diagramm zeigt die Dosis abhängige Wirkung von PLA-NP auf die γₘᵢₙ Werte von mit 1 Gew.-% rSP-C versetzten PLM-C (hellgraue Kreise). Die hellgrauen Kreise mit Punkt entsprechen der verbleibenden Konzentration von rSP-C in PLM-C in Abhängigkeit von der zugegebenen Menge an PLAM-NP. (Vergleichsbeispiel)

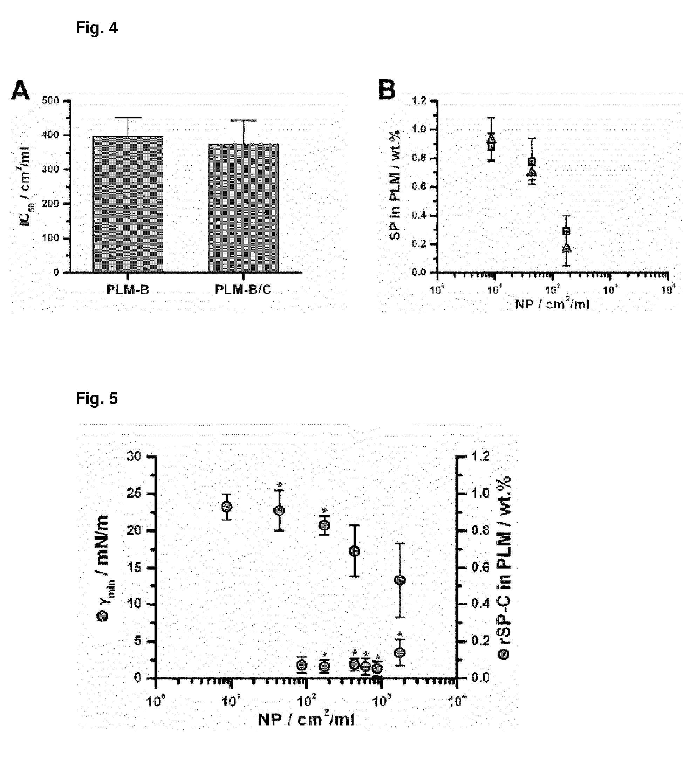

- Fig. 4A: Das Diagramm zeigt die biophysikalische Sensitivität des mit rSP-B oder mit rSP-B/C versetzten PLM gegenüber der Anwesenheit von polymeren NP. Das Diagramm zeigt den Einfluss von PLA-NP auf die γₘᵢₙ Werte von mit rSP-B bzw. rSP-B/C versetzten PLM in Form ermittelter IC₅₀ Werte für. (Vergleichsbeispiel)
- Fig. 4B: Die Grafik zeigt die Dosis-Wirkung von PLAM-NP auf den Gehalt an rSP-B (Quadrate) bzw. rSP-B/C (Dreiecke) in PLM nach erfolgter Inkubation. (Vergleichsbeispiel)
- Fig. 5: Gezeigt ist eine Dosis-abhängige Wirkung von PLA-NP-407 auf die γₘᵢₙ Werte und von PLAM-NP-407 auf den verbleibenden Gehalt an rSP-C in PLM-C.

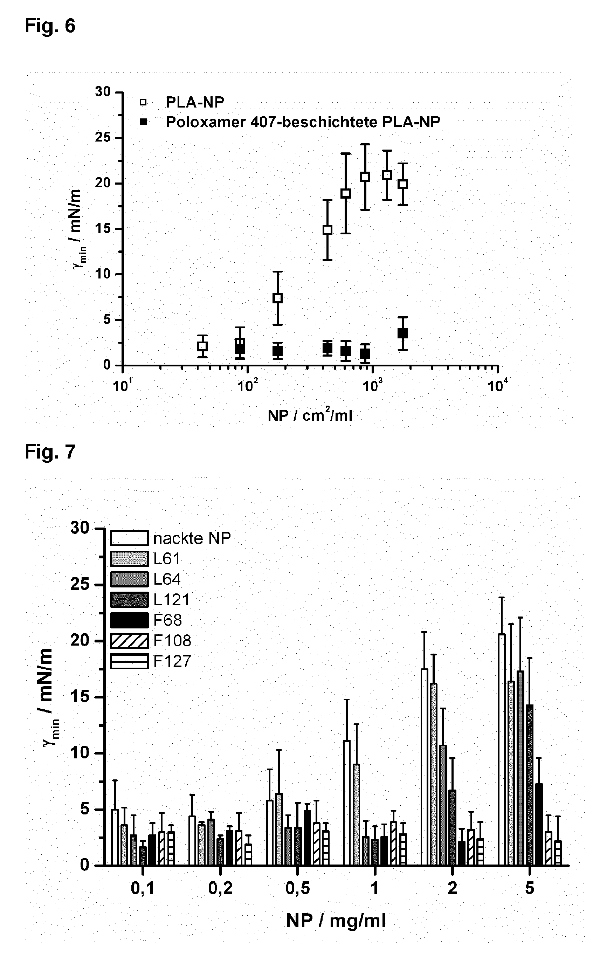

- Fig. 6: Die Grafik zeigt einerseits eine Dosis abhängige Wirkung von unbeschichtetem PLA-NP auf die γₘᵢₙ Werte des mit 1,0 Gew.-% rSP-C versetzten PLM hervor (offene Quadrate). Insbesondere ist auch hier erkennbar, dass die Höhe der γₘᵢₙ Werte der mit rSP-C versetzten PLM über den gesamten getesteten Konzentrationsbereich für hinzugefügte mit Poloxamer 407 beschichtete polymere NP, hier PLA-NP-F407, unbeeinflusst bleiben (schwarze Quadrate).
- Fig.7: Das Diagramm zeigt, dass für verschiedene Oberflächenbeschichtungen der NP eine Verbesserung der Oberflächenaktivität erreicht werden kann. Mit steigender Menge an unbeschichtetem (nacktem) NP ist eine Zunahme der γₘᵢₙ Werte und somit eine Abnahme der Oberflächenaktivität von Alveofact^{®} (2 mg/ml) zu erkennen (offene Balken). Zur Oberflächenbeschichtung der NP werden verschiedene Poloxamere verwendet und getestet (Balken mit Farbe oder Muster). Es wird deutlich, dass die Höhe der γₘᵢₙ Werte des Alveofacts^{®} für hinzugefügte beschichtete polymere NP wesentlich verringert werden kann.

### Materialien

Die in den nachfolgend beschriebenen Vergleichsbeispielen und Ausführungsbeispielen verwendeten Verbindungen 1,2-Dipalmitoyl-sn-glycero-3-phosphocholin (DPPC), 1-Palmitoyl-2-oleoyl-snglycero-3-phospho-(1'-*rac*-glycerol) (Natriumsalz) (POPG), Palmitinsäure (PA), superparamagnetische Eisenoxid Nanopartikel (NP) ("superparamagnetic iron oxide nanoparticles") (SPION, Größe von etwa 10 nm), Dextran (relatives Molekulargewicht von etwa 100 kDa), Poloxamer 407 (Molekulargewicht von etwa 12,2 kDa) und Poly(D,L-lactid) (PLA) sind kommerziell erhältlich.

### Methoden

### 1. Herstellung und Charakterisierung der Nanopartikel

Die Nanopartikel PLA-NP und SPION-beladene PLA-NP (PLAM-NP) wurden mit Hilfe der Lösungsmittel-Verdampfungs-Methode hergestellt wie sie aus Beck-Broichsitter M, Schmehl T, Gessler T, Seeger W, Kissel T: Development of a biodegradable nanoparticle platform for sildenafil: Formulation optimization by factorial design analysis combined with application of charge-modified branched polyesters. J Controlled Release 2012, 157:469-477 bekannt ist. Dazu wird mit oder ohne hinzugesetztes SPION (10 Gew.-%) PLA in Methylenchlorid gelöst (Gesamtfestgehalt von 25 mg/ml). Anschließend wurde die organische Phase (2 ml) in SDS (0.2 Gew.-%) enthaltendes filtriertes, doppelt-destilliertes Wasser (10 ml) überführt. Nach anfänglichem Vor-Mischen der zwei Phasen mit einem Homogenisator wurde die Emulsion über eine Spitze mit Ultraschall behandelt. Das organische Lösungsmittel wurde mittels Rotationsverdampfung entfernt. Die erhaltene kolloidale Dispersion wurde in einen Dialysebeutel überführt und gegen filtriertes, doppelt-destilliertes Wasser dialysiert. Nach Aufreinigung wurden die polymeren Nanopartikel durch Dialyse aufkonzentriert, wobei das Gegen-Dialysemedium aus filtriertem, doppelt-destilliertem Wasser enthaltend Dextran besteht. Abschließend wurden die Nanopartikel filtriert.

Die Massenkonzentration und Dichte der Nanopartikel in Suspension wurde nach Lyophilisierung mit Hilfe eines oszillierenden Dichtemessgerätes (DMA 4100 M, Anton Paar, Graz, Österreich) bestimmt. Die Größe und Größenverteilung, wie beispielsweise der Polydispersitätsindex (polydispersity index, PDI), der Nanopartikel wurden mittels dynamischer Lichtstreuung (dynamic light scattering, DLS) gemessen und das ζ-Potential wurde durch Laser-Doppler-Anemometrie (LDA) unter Verwendung eines Zetasizers NanoZS/ZEN3600 (Malvern Instruments, Herrenberg, Deutschland) bestimmt.

### 2. Beschichtung der Nanopartikel mit Poloxamer

Eine Suspension enthaltend die hergestellten Nanopartikel wurde für 12 Stunden bei 25°C mit einem Beschichtungs-Polymer, beispielsweise Poloxamer 407, einem Tensid, (Endkonzentration 0,1 Gew.-%) inkubiert. Das Beschichtungs-Polymer wird zum Zweck der Oberflächenbeschichtung nach diesem Ausführungsbeispiel adsorptiv auf Nanopartikel aufgebracht. Nach der Inkubation wurden die erhaltenen die oberflächenbeschichteten NP von restlichem Tensid durch wiederholte Zentrifugations- und Dispersionsrunden gereinigt und dann filtriert (5,0 µm)..

### 3. Bestimmung der Schichtdicke der adsorbierten Poloxamerschicht auf den NP

Die Schichtdicke (δ) der adsorbierten Poloxamer 407-Schicht auf der Oberfläche der Nanopartikel wurde mittels DLS-Messungen bestimmt. Diese Untersuchungen sind dem Fachmann geläufig (Besheer A, Vogel J, Glanz D, Kressler J, Groth T, Mäder K: Characterization of PLGA nanospheres stabilized with amphiphilic polymers: hydrophobically modified hydroxyethyl starch vs pluronics. Mol Phamaceutics 2009, 6:407-415; Beck-Broichsitter M, Kleimann P, Gessler T, Seeger W, Kissel T, Schmehl T: Nebulization performance of biodegradable sildenafil-loaded nanoparticles using the Aeroneb® Pro: Formulation aspects and nanoparticle stability to nebulization. Int J Pharm 2012, 422:398-408). Beim vorliegenden Ausführungsbeispiel wird die Schichtdicke δ abgeleitet aus einem Vergleich zwischen der Größe von unbeschichteten (*size₀*) und beschichteten (*size_{ads}*) Nanopartikeln (NP) (δ = (*size_{ads}* - *size₀*)/2).

### 4. Bereitstellung des Surfactant-Proteins B (SP-B)

SP-B (bovin) wurde aus einem natürlichen Surfactant der Rinderlunge (Alveofact®, Lyomark, Oberhaching, Germany) mit Hilfe der LH60-Chromatographie nach einem bekannten Verfahren isoliert (Curstedt T, Joernvall H, Robertson B, Bergman T, Berggren P: Two hydrophobic low-molecular-mass protein fractions of pulmonary surfactant. Characterization and biophysical activity. Eur J Biochem 1987, 168:255-262; Hawgood S, Benson BJ, Schilling J, Damm D, Clements JA, White RT: Nucleotide and amino acid sequences of pulmonary surfactant protein SP 18 and evidence for cooperation between SP 18 and SP 28-36 in surfactant lipid adsorption. Proc Natl Acad Sci USA 1987, 84:66-70; Markart P, Ruppert C, Grimminger F, Seeger W, Günther A: Fibrinolysis-inhibitory capacity of clot-embedded surfactant is enhanced by SP-B and SP-C. Am J Physiol Lung Cell Mol Physiol 2003, 284:L69-L76.).

### 5. Präparation der Surfactant Komponenten

Ein kommerziell erhältliches synthetisches Surfactant-Präparat wurde als Pulver zur Verfügung gestellt, welches DPPC (63,4 Gew.-%), POPG (27,8 Gew.-%), PA (4,5 Gew.-%), CaCl₂ (2,5 Gew.-%) und rekombinantes SP-C (rSP-C, 1,8 Gew.-%) enthält (Tabelle 1). Auf dieser Basis wurde die PLM-C Präparation hergestellt.

Synthetische pulmonale Surfactant Präparationen PLM-B und PLM-B/C mit 1,8 Gew.-% SP-B bzw. 1,8 Gew.-% SP-B/C wurden hergestellt durch Zugabe von bovinem SP-B zu entweder einer Phospholipid-Mischung (PLM) bzw. zu dem oben genannten synthetischen Surfactant-Präparat.

Die Phospholipid-Mischung PLM wurde bereitgestellt durch Lösung von DPPC, POPG, PA und CaCl₂ in einer Mischung von Chloroform/Methanol (2/1, v/v). Der Zusatz von SP-B zur Phospholipid-Mischung PLM zum Erhalt von PLM-B wurde erreicht durch Zugabe von hydrophobem SP-B gelöst in Chloroform/Methanol. PLM-B/C wurde durch Hinzufügung von SP-B zu dem oben genannten synthetischen Surfactant-Präparat erhalten.

Die Proben wurden dann unter Stickstoffgas getrocknet. Die Surfactant Präparationen wurden resuspendiert in mit Ca²+ versetzter mit NaCl-Lösung mit finaler PL-Konzentration von 50 mg/ml.

### 6. Inkubation polymerer NP mit pulmonalen Surfactants

Die zu testenden synthetischen pulmonalen Surfactant Präparationen wurden mit polymeren NP Suspensionen kombiniert, sodass die gewünschte finale Konzentration an PL, beispielsweise 2 mg/ml, und an polymerer NP in isotonischer NaCl-Lösung mit 2 mM Ca²+ eingestellt wird. Die Mengen an insgesamt zugesetzten polymeren NP werden dabei vorzugsweise basierend auf der Gesamtoberfläche bestimmt und nicht auf Grund der Massen. Die durch die polymeren NP eingebrachte Gesamtoberfläche wird auf einen Wert zwischen 9 cm²/ml (0,01 mg PLA-NP pro ml) und 4,355 cm²/ml (5,0 mg PLA-NP pro ml) eingestellt. Die Proben wurden gemischt und anschließend für 60 min bei 37°C inkubiert.

### 7. Biophysikalische Untersuchungen

Die Oberflächenaktivität von Proben wurde mit Hilfe der oszillierenden Blase Technik (oscillating bubble technique) untersucht (Electronetics Corp., Amherst, USA). Dieses Verfahren ist dem Fachmann bekannt (Beck-Broichsitter et al., 2011; Beck-Broichsitter et al., 2014; Seeger W, Grube C, Günther A, Schmidt R: Surfactant inhibition by plasma proteins: differential sensitivity of various surfactant preparations. Eur Respir J 1993, 6:971-977; Enhorning G: Pulsating bubble technique for evaluating pulmonary surfactant. J Appl Physiol 1977, 43:198-203; Seeger et al., 1992).

Alle Messungen wurden bei konstanter PL-Konzentration (2 mg/ml) in 2 mM Ca²⁺ enthaltender isotonischer NaCl-Lösung bei 37°C nach dem Inkubationsschritt durchgeführt. Dazu wurden Proben von 35 µl in eine Einwegprobenkammer überführt und Blasen-Pulsieren wurde durch sinusförmiges Oszillieren des Blasenradius (*r*) zwischen 0,4 und 0,55 mm eingeleitet. Die Periode betrug 20 Zyklen pro Minute. Die Druckdifferenzen (Δ*p*) über die Luft/Flüssigkeits-Grenzfläche wurden kontinuierlich aufgezeichnet. Die Oberflächenspannung (y) wurde dann über die Young-Laplace Gleichung (γ = (Δ*p* * r)/2) berechnet. Die Werte für die minimale Oberflächenspannung (γₘᵢₙ) wurden nach 300 sec abgelesen. Dosis-Wirkung Kurvencharakteristika, wie beispielsweise die halbmaximale biophysikalische inhibitorische polymere NP Konzentration (IC₅₀ Wert) wurden nach der sigmoidalen Dosis-Wirkungs-Kurve berechnet.

### 8. Bestimmung des SP-Gehalts nach Behandlung (challenge) des pulmonalen Surfactants mit polymeren NP

Eine Verringerung des SP-Gehalts in der Surfactant Präparation, verursacht durch Zugabe von polymeren NP, wurde untersucht mit pulmonalen Surfactants jeweils enthaltend 1 Gew.-% SP-B, rSP-C oder SP-B/C.

Präparationen von synthetischen pulmonalen Surfactants und polymeren NP, insbesondere PLAM-NP und PLAM-NP 407, wurden kombiniert um die gewünschte finale Konzentration an PL, also hier 2 mg/ml, und die Konzentration von polymeren NP in 2 mM Ca²+ enthaltender isotonischer NaCl zu erhalten. Die Proben wurden gemischt. Nach einem Inkubationsschritt von 60 min bei 37°C wurden die NP magnetisch von den Surfactant Proben getrennt. Der Überstand wurde entfernt und der SP-Gehalt nach der dem Fachmann bekannten Methode nach Bradford bestimmt.

### Ergebnisse

### 1. Charakterisierung pulmonaler Surfactants (Vergleichsbeispiel)

Die biochemische Zusammensetzung und die biophysikalische Charakterisierung der gemäß den oben dargestellten Ausführungsbeispielen hergestellten synthetischen pulmonalen Surfactant Päparationen gehen aus der Tabelle 1 hervor. Zu erkennen sind die Werte für PLM aufweisend 1,8 Gew.-% SP-B (PLM-B), aufweisend rekombinantes SP-C (rSP-C) (PLM-C) und aufweisend SP-B und rSP-C (PLM-B/C). Das PLM enthält jeweils ein Phosphatidylcholin (hier DPPC), ein Phosphatidylglycerol (hier POPG), eine Fettsäure (hier PA), und CaCl₂. Die Werte für γₘᵢₙ/mN/m sind als Mittel mit Standardabweichung (n=5) angegeben.

**Tabelle 1. Biochemische Zusammensetzung und biophysikalische Charakterisierung der synthetischen pulmonalen Surfactant Päparationen.**

| **Präparation** | **PL (Gew.-%)** | **SP (Gew.-%)** | **Verschiedenes (Gew.-%)** | **γₘᵢₙ/mN/m** |
|---|---|---|---|---|
| PLM-B | DPPC (63,4) | SP-B (1,8) | PA (4,5) | 3,3 ± 2,0 |
| | POPG (27,8) | | CaCl₂ (2,5) | |
| PLM-C | DPPC (63,4) | rSP-C (1,8) | PA (4,5) | 2,5 ± 1,7 |
| | POPG (27,8) | | CaCl₂ (2,5) | |
| PLM-B/C | DPPC (63,4) | SP-B (0,45) | PA (4,5) | 2,9 ± 1,6 |
| | POPG (27,8) | rSP-C (1,35) | CaCl₂ (2,5) | |

Die biophysikalischen Eigenschaften der synthetischen Surfactants wurden mit Hilfe der oszillierenden Blase Technik (oscillating bubble technique) bei konstanter PL-Konzentration von 2 mg/ml bestimmt.

Alle getesteten Präparationen zeigen hervorragende biophysikalische Aktivität, also eine niedrige Oberflächenspannung, mit Werten für γₘᵢₙ/mN/m von ca. 3 mN/m (siehe Tabelle 1).

Weiterhin kann mit Fig. 1 gezeigt werden, dass in den verschiedenen PLM Präparationen eine Verringerung des SP Gehalts einen Dosis-abhängigen Anstieg der γₘᵢₙ Werte bewirkt. Man erkennt in Fig. 1 eine Auswirkung des SP-Gehaltes an SP-B (offene Quadrate), rSP-C (offene Kreise) und SP-B/C (offene Dreiecke) im jeweiligen PLM auf die Oberflächenspannung, also die dargestellten γₘᵢₙ Werte. Die Werte sind als Mittel mit Standardabweichung (n=5) angegeben. Das eingegrenzte Rechteck gibt das Mittel mit Standardabweichung für reines PLM wieder. Es ist deutlich zu erkennen, das in dem hier verwendeten Modell die Anwesenheit der verschiedenen SP Proteine zu einer Disis-abhängigen Verringerung der Oberflächenspannung führt bzw. umgekehrt eine Verringerung des SP Gehalts einen Dosis-abhängigen Anstieg der γₘᵢₙ Werte zur Folge hat. Dies unterstreicht die wichtige Funktion der Surfactant-assoziierten Proteine im pulmonalen Surfactant der Lunge bei der Aufrechterhaltung einer niedrigen

Oberflächenspannung. Alle hier untersuchten mit SP versetzten PLM zeigen überlagerte Dosis-Wirkungs-Kurven Charakteristika, insbesondere γₘᵢₙ Werte von > 5 mN/m werden bei SP-Konzentrationen von < 0,1-0,2 Gew.-% erreicht. Entsprechend zeigen SP-freie PLM gemäß dem in Fig. 1 eingezeichneten eingerahmten Rechteck γₘᵢₙ Werte von ca. 20 mN/m.

Die Anwesenheit der SP Komponenten SP-B, rSP-C und SP-B/C verbessert somit die unzureichende Oberflächenaktivität der PLM allein ohne SP. Für SP Konzentrationen überschreitend 0,1 - 0,2 Gew.-% SP kann eine maximale Fähigkeit zur Verringerung der dynamischen Oberflächenspannung, also Werte für γₘᵢₙ von < 5 mN/m festgestellt werden. Diese Werte für γₘᵢₙ in dem hier etablierten Modell spiegeln sehr gut die *in vivo* gemessenen Werte für das pulmonale Surfactant wider (vgl. Zuo *et al.,* 2008).

### 2. Charakterisierung polymerer NP

Die physikochemischen Eigenschaften der hergestellten polymeren Nanopartikel (mit und ohne Oberflächenbeschichtung) sind in Tabelle 2 zusammengefasst.

**Tabelle 2. Physikochemische Eigenschaften der polymeren Nanopartikel**

| Nanopartikel | Größe nm | PDI | ζ-Potential mV | δ^{a} nm | γₘᵢₙ mN/m |
|---|---|---|---|---|---|
| PLA-NP | 65,3 ± 3,2 | 0,073 ± 0,012 | -44,3 ± 2,5 | n.b. | 70,7 ± 0,9 |
| PLA-NP-407 | 75,9 ± 2,6 | 0,053 ± 0,008 | -23,6 ± 0,8 | 5,3 ± 0,8 | 64,3 ± 1,6 |
| PLAM-NP | 70,5 ± 4,9 | 0,068 ± 0,016 | -44,4 ± 3,1 | n.b. | n.b. |
| PLAM-NP-407 | 83,1 ± 4,1 | 0,061 ± 0,012 | -24,5 ± 2,1 | 6,3 ± 0,8 | n.b. |

| | | | | | |
|---|---|---|---|---|---|
| ^{a} adsorbierte Poloxamer 407 Schichtdicke n.b. = nicht bestimmt | | | | | |

Die Werte sind als Mittel mit Standardabweichung (n ≥ 4) angegeben.

Die nach der oben beschrieben Methode hergestellten unbeschichteten PLA Nanopartikel zeigen eine mittlere Größe von 65,3 ± 3,2 nm mit enger Größenverteilung (PDI < 0,1). Das korrespondierende ζ-Potential beträgt -44,3 ± 2,5 mV. Beschichtung der PLA NP mit einem Poly(ethylenglycol)-*block-*Poly(propylenglycol)-*block*-Poly(ethylenglycol) (PEG-*b*-PPG-*b*-PEG) Triblock Copolymer (also ein Poloxamer, hier Poloxamer 407) (PLA-NP-407) bewirkt eine Vergrößerung der NP auf ca. 76 nm. Folglich beträgt die Schichtdicke δ der adsorbierten Poloxamer 407 Schicht auf der Oberfläche der polymeren NP 5,3 ± 0,8 nm. Außerdem zeigt PLA-NP-407 eine erhebliche Veränderung des ζ-Potentials (-23,6 ± 0,8 mV). Es wird deutlich, dass die anzustrebende Annäherung des ζ-Potentials für die erfindungsgemäßen polymeren Nanopartikelformulierungen an 0 mV erreicht wird.

Unbeschichtete magnetische PLA-NP (PLAM-NP) wurden hergestellt, um den Einfluss der polymeren NP auf die Surfactant Zusammensetzung zu untersuchen. Diese PLAM-NP weisen eine Größe von 70,5 ± 4,9 nm, ein PDI von < 0,1 und ein ζ-Potential von -44,4 ± 3,1 mV auf. Vergleichbar zu den für beschichtete PLA-NP, also die PLA-NP-407, beobachteten physikochemischen Veränderungen, sind verursacht durch die adsorbierte Poloxamer 407 Schicht auch für die PLAM-NP-407 eine gesteigerte NP Größe, ca. 83 nm und ein verschobenes ζ-Potential von -24,5 ± 2,1 mV festzustellen. Hierbei beträgt die Schichtdicke δ der adsorbierten Poloxamer 407-Schicht auf der Oberfläche der NP etwa 6 nm.

Die Oberflächenaktivität wurde für die verschiedenen NP an der pulsierenden Blase wie oben ausgeführt bestimmt. Dabei konnte kein relevanter Abfall der Oberflächenspannung durch die Poloxamer Oberflächenbeschichtung festgestellt werden. So bleibt der Wert für γₘᵢₙ gemäß Tabelle 2 bei > 64 mN/m.

Die physikochemischen Eigenschaften der hier verwendeten NP entsprechen somit denen von häufig genutzten nanoskaligen Materialien als Arzneistoffträger für die pulmonale Applikation.

### 3. Biophysikalische Inhibition des pulmonalen Surfactants durch polymere NP (Vergleichsbeispiele)

Mit den nachfolgenden Vergleichsbeispielen wird das Ausmaß der Inhibition des pulmonalen Surfactants durch polymere NP gezeigt. Dazu wird hier die Sensitivität von synthetisch hergestellten PLM getestet, wobei unterschiedliche Mengen, insbesondere 0,2-1,5 Gew.-%, und verschiedene Arten von SP, hier B, C und B/C, eingesetzt werden. Die Surfactant Präparationen werden mit verschiedenen Mengen unbeschichteten PLA-NP inkubiert. Die Mengen der eingesetzten unbeschichteten PLA-NP werden hier in Gesamtoberfläche pro Volumen in cm²/ml angegeben. Die biophysikalischen Eigenschaften wurden dann bei einer konstanten PL Konzentration von 2 mg/ml in Versuchsanordnungen an der oszillierenden Blase bestimmt.

Gemäß den Fig. 2, Fig. 3 und Fig. 4A zeigen die Messungen der Oberflächenaktivität dabei deutlich einen Dosis abhängigen Verlust der Eigenschaft zur Verringerung der dynamischen Oberflächenspannung für die getesteten Präparationen nach Zugabe polymerer NP. Die Sensitivität der einzelnen getesteten Präparationen hängt dabei auch vom Gehalt und von der Art der des verwendeten SP ab.

Aus dem in Fig. 2A dargestellten Diagramm geht eine Dosis abhängige Wirkung von PLA-NP auf die γₘᵢₙ Werte des mit 0,2 Gew.-% (hellgraue Kreise) bzw. 1,5 Gew.-% (dunkle Kreise) rSP-C versetzten des PLM hervor. Die durchgezogenen Linien repräsentieren dabei eine sigmoidale Dosis-Wirkung basierend auf den experimentellen Daten. Man erkennt aus den Figuren 2A und 3, dass eine Erhöhung des rSP-C Gehalts die Stabilität des PLM-C gegenüber einer polymeren NP induzierten biophysikalischen Inhibition verbessert.

In Fig. 2B ist ein Zusammenhang zwischen der rSP-C Konzentration in PLM und der PLA-NP induzierten biophysikalischen Inhibition gezeigt, wobei die IC₅₀ Werte aus der sigmoidale Dosis-Wirkung gemäß Fig. 2A errechnet wurden. Das Maß der aus den Dosis-Wirkung Kurven gemäß Fig. 2A in individuellen Inhibitions-Ansätzen abgeleiteten biophysikalischen Inaktivierung (IC₅₀) wird gemäß Fig. 2B in einem Diagramm gegen die Menge des im PLM anwesenden rSP-C aufgetragen. Die IC₅₀ entspricht dabei jeweils der halbmaximalen biophysikalisch inhibitorischen Konzentration der polymeren NP.

Auch in Fig. 2B ist klar zu erkennen, dass mit steigender Konzentration an rSP-C die Fähigkeit der getesteten Surfactant Präparationen zur Verringerung der dynamischen Oberflächenspannung verbessert wird. Mit anderen Worten, mit steigender Konzentration an rSP-C im PLM ist eine höhe Menge an PLA-NP tolerierba, ohne dass die Fähigkeit zur Verringerung der dynamischen Oberflächenspannung für die getesteten Surfactant Präparationen nach Zugabe polymerer NP verloren geht. Die in Fig. 2B eingezeichnete gerade Linie zeigt einen linearen Zusammenhang (Fit) für die experimentellen Daten (R² = 0,962). Die dunkle Raute repräsentiert den IC₅₀ Wert für negativ geladene Polystyren NP mit einem Durchmesser von etwa 100 nm inkubiert mit PLM-C mit 1,8 Gew.-% rSP-C. Die Werte sind als Mittel mit Standardabweichung (n ≥ 5) angegeben.

Fig. 3 zeigt einerseits wiederum die Dosis abhängige Wirkung von PLA-NP auf die γₘᵢₙ Werte von mit 1 Gew.-% rSP-C (hellgraue Kreise) versetzten PLM-C. Andererseits repräsentieren die hellgrauen Kreise mit Punkt die verbleibende Konzentration von rSP-C in PLM-C in Abhängigkeit von der zugegebenen Menge an PLAM-NP. Die Werte sind als Mittel mit Standardabweichung (n ≥ 5) angegeben. Man erkennt deutlich die Abnahme der rSP-C Menge in PLM-C mit steigender Menge an zugegebenem PLAM-NP.

Das in Fig. 4A dargestellte Vergleichsbeispiel betrifft die biophysikalische Sensitivität des PLM versetzt mit rSP-B oder mit rSP-B/C gegenüber der Anwesenheit von polymeren NP. Das Diagramm zeigt ermittelte Dosis-Wirkung-Kurven Charakteristika, nämlich in der Form von IC₅₀ Werten, für den Einfluss von PLA-NP auf die γₘᵢₙ Werte von mit 1 Gew.-% rSP-B bzw. mit 1 Gew.-% rSP-B/C versetzten PLM. Die IC₅₀ Werte wurden aus individuellen Inhibitionsansätzen unter Anwendung eines sigmoidalen Dosis-Wirkungs Fits für die gemessenen Daten ermittelt.

Für die getesteten PLM-B und PLM-B/C Präparationen konnten gemäß Fig. 4A gegenüber den in Fig. 2B für PLM-C gezeigten Daten leicht erhöhte IC₅₀ Werte festgestellt werden, wobei jedoch für alle drei Systeme durchaus vergleichbare Charakteristika gegeben sind.

Um das erhebliche Potential der polymeren unbeschichteten NP zur biophysikalischen Inaktivierung des Surfactants aufzuzeigen, wurden die synthetischen Surfactant Präparationen mit 1,0 Gew.-% SP-B, rSP-C bzw. SP-B/C jeweils mit PLAM-NP versetzt und inkubiert. Nach magnetischer Abtrennung der polymeren NP wird dann der SP Gehalt im Überstand analysiert werden. In Fig. 4B ist die Dosis-abhängige Wirkung von PLAM-NP auf den Gehalt an rSP-B (Quadrate) bzw. rSP-B/C (Dreiecke) in PLM nach erfolgter Inkubation dargestellt. Die Werte sind als Mittel mit Standardabweichung (n ≥ 5) angegeben. Man erkennt deutlich die Abnahme der rSP-B Menge bzw. der rSP-B/C Menge in den PLM-Ansätzen mit steigender Menge an zugegebenem PLAM-NP.

Es kann somit festgestellt werden, dass ein Anstieg der Menge an zugesetztem polymeren NP zu einer Dosis-abhängigen Verarmung des SP Gehalts in der resultierenden Surfactant Präparation führt (Fig. 3 und Fig. 4B). Insgesamt ist zu erkennen, dass die durch Zugabe von polymeren NP verursachte Inaktivierung der Oberflächeneigenschaften und Verarmung des SP Gehalts in synthetisch hergestellten Surfactant Präparationen (Fig. 2A, Fig. 3 und Fig. 4) in guter Übereinstimmung mit den Ergebnissen zur Oberflächenaktivität bei SP verarmten PLM gemäß Fig. 1 ist.

### 4. Biophysikalische Inhibition des pulmonalen Surfactants durch erfindungsgemäße polymere Nanopartikelformulierungen

Nach einem ersten erfindungsgemäßen Ausführungsbeispiel werden zur Vermeidung der durch polymere NP verursachten unerwünschten Inhibition des Surfactants die NP oberflächenbeschichtet. Im Rahmen des gezeigten Ausführungsbeispiels wird die Oberfläche mit einem PEG-b-PPG-b-PEG Triblock Copolymer (Poloxamer 407). Die wie oben dargestellt gewonnenen oberflächenbeschichteten polymeren NP werden gemäß Fig. 5 hinsichtlich ihrer beeinträchtigenden Wirkungen auf die biophysikalischen Eigenschaften und die Zusammensetzung eingesetzter Surfactant Präparationen untersucht.

In Fig. 5 ist die Dosis-abhängige Wirkung von PLA-NP-407 auf die γₘᵢₙ Werte und von PLAM-NP-407 auf den verbleibenden Gehalt an rSP-C in PLM-C dargestellt. Hierzu wurde jeweils PLM-C mit einer Menge von 1 Gew.-% rSP-C eingesetzt. Die Werte sind als Mittel mit Standardabweichung (n ≥ 5) angegeben. Statistisch signifikante Unterschiede mit p < 0,05 sind mit einem Stern (*) gekennzeichnet und liegen insbesondere für folgenden Vergleich vor: Poloxamer 407 beschichtete NP vs. unbeschichtete polymere NP (vgl. Daten in Fig. 3).

Die Oberflächenaktivität, also insbesondere die Höhe der γₘᵢₙ Werte, der mit rSP-C versetzten PLM ist nach Fig. 5 über den gesamten getesteten Konzentrationsbereich für hinzugefügte mit Poloxamer 407 beschichtete polymere NP, hier PLA-NP-407, unbeeinflusst. Außerdem sind substantiell höhere SP Konzentrationen nach PLM-C Inkubation mit PLAM-NP-407 im Vergleich zu PLAM-NP behandelten Proben feststellbar (vgl. Fig. 3).

Aus dem in Fig. 6 dargestellten Diagramm geht einerseits eine Dosis abhängige Wirkung von unbeschichtetem PLA-NP auf die γₘᵢₙ Werte des mit 1,0 Gew.-% rSP-C versetzten PLM hervor (offene Quadrate). Man erkennt wiederum, dass eine Erhöhung der Menge an unbeschichtetem PLA-NP (angegeben in cm²/ml) zu einem zu einem steigenden Verlust der Eigenschaft zur Verringerung der dynamischen Oberflächenspannung für die getesteten Präparationen führt, also einer Erhöhung des γₘᵢₙ Wertes. Insbesondere ist auch hier erkennbar, dass die Oberflächenaktivität, also hier die Höhe der γₘᵢₙ Werte, der mit rSP-C versetzten PLM über den gesamten getesteten Konzentrationsbereich für hinzugefügte mit Poloxamer 407 (Handelsname Pluoronic® F127) beschichtete polymere NP, hier PLA-NP-F407, unbeeinflusst bleiben (schwarze Quadrate).

Aus dem in Fig. 7 dargestellten Diagramm geht hervor, dass für verschiedene Oberflächenbeschichtungen der NP, hier Polystyrol-NP, eine Verbesserung der Oberflächenaktivität erreicht werden kann. Mit steigender Menge an unbeschichtetem (nacktem) NP ist eine Zunahme der γₘᵢₙ Werte und somit eine Abnahme der Oberflächenaktivität von Alveofact^{®} (2 mg/ml) zu erkennen (offene Balken). Im Rahmen der Ausführungsbeispiele gemäß Fig. 7 werden zur Oberflächenbeschichtung der NP Pluoronic® L61 (Poloxamer 181), L64, L121, F68 (Poloxamer 188), F108 (Poloxamer 338) und F127 (Poloxamer 407) verwendet und getestet (Balken mit Farbe oder Muster). Es wird deutlich, dass die Höhe der γₘᵢₙ Werte des Alveofacts^{®} für hinzugefügte beschichtete polymere NP wesentlich verringert werden kann. Dieser Effekt kann für alle hier getesteten Beschichtungen beobachtet werden.

Die Erfindung ist nicht auf eine der vorbeschriebenen Ausführungsformen beschränkt, sondern in vielfältiger Weise abwandelbar.

Sämtliche aus den Ansprüchen, der Beschreibung und den Zeichnungen hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

## Patentansprüche

1. Polymere Nanopartikelformulierung mit einem Durchmesser zwischen 10 nm und 1000 nm, wobei die polymere Nanopartikelformulierung im Inneren einen Kern aufweist und der Kern wenigstens ein Kern-Polymer umfasst, **dadurch gekennzeichnet, dass** auf der Oberfläche des Kerns eine maskierende Beschichtung vorgesehen ist, wobei die Oberflächenbeschichtung ein Beschichtungs-Polymer umfasst und eine Schichtdicke von 1 nm bis 20 nm aufweist.

2. Polymere Nanopartikelformulierung gemäß Anspruch 1, **dadurch gekennzeichnet, dass** das Beschichtungs-Polymer der Oberflächenbeschichtung kovalent oder adsorptiv auf den Kern aufgebracht ist.

3. Polymere Nanopartikelformulierung gemäß Anspruch 1 oder 2, **dadurch gekennzeichnet, dass** die Oberflächenbeschichtung eine Schichtdicke von 2 bis 10 nm, insbesondere 4 bis 10 nm, vorzugsweise 5 bis 8 nm, aufweist.

4. Polymere Nanopartikelformulierung gemäß einem der Ansprüche 1 bis 3, **dadurch gekennzeichnet, dass** die polymere Nanopartikelformulierung ein ζ-Potential von größer als -40 mV, insbesondere von größer als -30 mV, vorzugsweise von im Wesentlichen nahe 0 mV aufweist.

5. Polymere Nanopartikelformulierung gemäß einem der Ansprüche 1 bis 4, **dadurch gekennzeichnet, dass** das Kern-Polymer biokompatibel und bioabbaubar ist und vorzugsweise ausgewählt ist aus der Gruppe bestehend aus Polyestern, Poly(dioxanon)en, Poly(hydroxybutyrat)en, Poly(cyanoacrylat)en, Poly(amid)en, Poly(urethan)en, Poly(organophosphazen)en, Polyanhydriden, Polyorthoestern, Polycarbonaten, Polyketalen, Proteinen und Polysacchariden.

6. Polymere Nanopartikelformulierung gemäß einem der Ansprüche 1 bis 5, **dadurch gekennzeichnet, dass** das Kern-Polymer ein Polyester ist, das ausgewählt ist aus der Gruppe bestehend aus Poly(D,L-lactid) (PLA) und Polylactid-co-Glycolid (PLGA).

7. Polymere Nanopartikelformulierung gemäß einem der Ansprüche 1 bis 6, **dadurch gekennzeichnet, dass** das Beschichtungs-Polymer ausgewählt ist aus der Gruppe bestehend aus Homopolymer, Copolymer, Blockpolymer, Blockcopolymer, Pfropfcopolymer, Sternpolymer, Kammpolymer, hochverzweigtes Polymer, statistisches Polymer, Dendrimer und deren Mischungen.

8. Polymere Nanopartikelformulierung gemäß einem der Ansprüche 1 bis 7, **dadurch gekennzeichnet, dass** das Beschichtungs-Polymer ein Blockcopolymer, insbesondere ein Poloxamer, oder ein Polyether, insbesondere ein Polyethylenglycol (PEG), ist.

9. Polymere Nanopartikelformulierung gemäß einem der Ansprüche 1 bis 8, **dadurch gekennzeichnet, dass** die polymere Nanopartikelformulierung mit wenigstens einem Wirkstoff und/oder Diagnostikum beladen ist.

10. Polymere Nanopartikelformulierung gemäß einem der Ansprüche 1 bis 9, **dadurch gekennzeichnet, dass** die polymere Nanopartikelformulierung einen zur pulmonalen Applikation geeigneten Wirkstoff und/oder geeignetes Diagnostikum enthält.

11. Arzneimittel enthaltend eine polymere Nanopartikelformulierung gemäß einem der Ansprüche 1 bis 10.

12. Polymere Nanopartikelformulierung gemäß einem der Ansprüche 1 bis 10 zur Verwendung bei der Behandlung von respiratorischen Erkrankungen.

13. Polymere Nanopartikelformulierung gemäß einem der Ansprüche 1 bis 10 zur Verwendung zur Prävention und/oder Behandlung von an einer respiratorischen Erkrankung leidenden Patienten, insbesondere solcher Erkrankungen, die mit einem Anstieg der Oberflächenspannung in der Lunge und einer Schädigung des pulmonalen Surfactants einhergehen.

14. Polymere Nanopartikelformulierung zur Verwendung zur Prävention und/oder Behandlung von an einer respiratorischen Erkrankung leidenden Patienten gemäß einem der Ansprüche 12 bis 13, wobei nach pulmonaler Verabreichung der polymeren Nanopartikelformulierung die minimale Oberflächenspannung (γₘᵢₙ Werte) des pulmonalen Surfactant-Systems des Patienten kleiner als 10 mN/m, vorzugsweise kleiner als 5 mN/m, und/oder die Konzentration von Surfactant-assoziierten Proteinen (SP) höher als 0,1 Gew.-%, vorzugsweise höher als 0,2 Gew.-%, ist.

15. Polymere Nanopartikelformulierung zur Verwendung zur Prävention und/oder Behandlung von an einer respiratorischen Erkrankung leidenden Patienten gemäß einem der Ansprüche 12 bis 13, wobei die Patienten ausgewählt sind aus einer Gruppe, die dadurch charakterisiert ist, dass die minimale Oberflächenspannung (γₘᵢₙ Werte) des pulmonalen Surfactant-Systems des Patienten größer als 5 mN/m, vorzugsweise größer als 10 mN/m, und/oder die Konzentration von Surfactant-assoziierten Proteinen (SP) niedriger als 0,2 Gew.-%, vorzugsweise niedriger als 0,1 Gew.-%, ist.
